# EUROPÄISCHE PATENTSCHRIFT

(11) **EP 3 968 871 B1**
(45) Veröffentlichungstag und Bekanntmachung des Hinweises auf die Patenterteilung: **02.07.2025**
(21) Anmeldenummer: 20726787.3
(22) Anmeldetag: 15.05.2020
(51) Int. Cl.: A61B 17/15, A61B 17/17, A61B 34/10

(54) **MEDIZINTECHNISCHE POSITIONIER-/AUSRICHTVORRICHTUNG MIT FÜHRUNGSSCHABLONE UND OPTISCHER MARKIERUNGSEINRICHTUNG**
MEDICAL TECHNOLOGY POSITIONING/ALIGNMENT DEVICE WITH GUIDE TEMPLATE AND OPTICAL MARKING DEVICE
ARRANGEMENT DE POSITIONNEMENT/ORIENTATION MÉDICO-TECHNIQUE AVEC GABARIT DE GUIDAGE ET DISPOSITIF DE MARQUAGE

(30) Priorität: 16.05.2019 DE 102019112898
(43) Veröffentlichungstag der Anmeldung: 23.03.2022
(73) Patentinhaber: Aesculap AG, 78532 Tuttlingen (DE)
(72) Erfinder: MARIE, Vincent, 78234 Engen (DE)
(74) Vertreter: Winter, Brandl - Partnerschaft mbB
(86) Internationale Anmeldenummer: PCT/EP2020/063657
(87) Internationale Veröffentlichungsnummer: WO 2020/229673

(56) Entgegenhaltungen:
- FR-A1- 2 917 284
- US-A1- 2009 234 360
- US-A1- 2010 087 829
- US-A1- 2018 168 826

## Beschreibung

Die vorliegende Erfindung betrifft eine medizintechnische Vorrichtung zur Positionierung und/oder Orientierung eines medizinischen Instruments zur endoprothetischen Versorgung oder eines Implantats relativ zu einem Patienten unter direkter Visualisierung/Projektion einer Instrumenten- oder Implantats-spezifischen und dessen Positionierung und/oder Orientierung betreffenden geometrischen Information an bzw. auf dem Patienten.

### Hintergrund der Erfindung

Bei vielen chirurgischen Anwendungen, insbesondere bei muskulo-skelettalen Anwendungen wie der Orthopädie, ist ein chirurgisches/medizintechnisches Instrument wie Knochensäge, Bohrer, Fräser, etc. oder Implantat relativ zum Patienten an verschiedenen Strukturen und/oder Orientierungspunkten wie Knochen auszurichten. In diesem Zusammenhang ist derzeit bekannt, das Instrument oder Implantat zunächst manuell auszurichten und die Ausrichtung nachfolgend mit einer mechanischen Hilfseinrichtung, wie zum Beispiel extra-medullären Ausrichtungsstangen oder Platten zur Prüfung von Resektionsdicken, zu überprüfen. Dabei spielt eine visuelle Begutachtung der jeweils vorliegenden Situation durch den Operateur eine entscheidende Rolle, so dass die Qualität der Ausrichtung teilweise wesentlich von der Erfahrung und Sorgfalt des Operateurs abhängt.

Ein häufiger Fehler bei auf Augenmaß basierten Ausrichtungen von Instrumenten/Implantaten relativ zum Patienten/Patientenknochen besteht darin, Winkel und/oder Parallelen zwischen zwei Objekten (Instrument versus Knochen) selbst bei vollständiger Sichtbarkeit der beiden Objekte falsch zu bewerten oder Parallaxenfehler aufgrund von Perspektiven oder Projektionen zu erzeugen, die in falsche Richtungen oder an umhüllten und/oder unebenen Oberflächen wahrgenommen werden.

Eine visuelle Begutachtung zur Ausrichtung von chirurgischen/medizintechnischen Instrumenten und/oder Implantaten relativ zum Patienten/Patientenknochen wird jedoch auch in Situationen angewendet, bei denen an der Ausrichtung beteiligte Strukturen teilweise für den Operateur nicht direkt sichtbar sind, sondern sich zum Beispiel an einer Knochenoberfläche befinden, die durch Gewebe verdeckt ist und nicht oder nur zum Teil sichtbar ist. Ein dabei oftmals auftretendes Problem ist wiederum oftmals eine Erzeugung eines Parallaxenfehlers aufgrund einer fehlerhaften Erkennung von Perspektiven oder Projektionen, zum Beispiel in Folge von Richtungsfehlern.

Die vorstehend beschriebene Problematik tritt insbesondere häufig während TKA-Operationen (Total-Endprothesen) während der Ausrichtung von Schnittführungen für proximale Tibia-Schnitte oder distale Femur-Schnitte auf. In der manuellen Chirurgie ohne Verwendung eines Navigationssystems werden derartige Schnittführungen mit Hilfe von internen oder in dem Fall der Tibia externen Ausrichtungssystemen positioniert und ausgerichtet. Interne Ausrichtungssysteme sind normalerweise relativ zum Knochenmark referenziert und nicht sichtbar. Bei externen Ausrichtsystemen gibt es zusätzlich einige Daumenregeln. Ein Zwischenraum von zwei bis drei Fingerbreiten zwischen dem Ausrichtungssystem und dem Knochen des Patienten sollte für eine gute Ausrichtung sorgen. Alle vorgenannten Lösungen beinhalten jedoch die Problematik von Parallaxen-Fehlern und schlechter Perspektive.

### Stand der Technik

Bislang gibt es kein System auf dem Markt, mit dem sich Ausrichtungsachsen von chirurgischen Instrumenten oder Implantaten wie insbesondere Operationsinstrumenten am Patienten, insbesondere auf dessen Haut, Knochen oder auf einem Verband des Patienten, darstellen und/oder verkörpern lassen, um eine Positionierung der Instrumente oder Implantate relativ zum Patienten/Patientenknochen und insbesondere relativ zum Operationsgebiet mit hoher Genauigkeit vorzugeben und/oder zu überprüfen. Bekannte Systeme und Lösungen beruhen in der Regel auf externen mechanischen Stäben oder Platten zur Überprüfung der Positionierung und Ausrichtung der Instrumente.

Ein weiterer Nachteil ist, dass sich einige Informationen mit herkömmlicher Technologie nicht darstellen lassen. Es gibt insbesondere keine Möglichkeit, eine Achse oder Schnittebene direkt am Knochen des Patienten zu visualisieren. Gegenwärtige Instrumentensysteme bieten keine verlässliche Kontrolle über die genaue Positionierung des Instruments im Operationsfeld.
Aus der US 2018/0168826 A1 ist eine medizintechnische Positionier- und/oder Ausrichtungsvorrichtung mit einer bezüglich eines Patientenknochens positionier-, ausricht- und am Patientenknochen temporär fixierbaren Führungsschablone für ein chirurgisches Instrument, welche einen Führungsabschnitt zur Führung des chirurgischen Instruments entsprechend der Position und Ausrichtung der Schablone bezüglich des Patientenknochens aufweist, und mit einer optischen Markierungseinrichtung, welche zumindest eine Lichtquelle hat, bekannt. Aus der FR 2 917 284 A1, der US 2010/087829 A1 und der US 2009/234360 A1 ist eine medizintechnische Positionier- und/oder Ausrichtungsvorrichtung gemäß dem Oberbegriff des Anspruchs 1 bekannt.

Vor diesem Hintergrund liegt der vorliegenden Erfindung die Aufgabe zugrunde, die genannten Nachteile des Stands der Technik zu verringern, insbesondere eine Vorrichtung zu schaffen, mit der die Benutzerfreundlichkeit bei einer Positionierung von Operationsinstrumenten insbesondere einer Knochensäge oder von Implantaten unter größtmöglicher Ergonomie und ohne Qualitätseinbußen maximiert werden kann.

Diese Aufgabe wird durch eine medizinische Positionier-/Ausrichtvorrichtung gemäß Anspruch 1 sowie eine optische Markierungseinrichtung für eine solche medizinische Positionier-/Ausrichtvorrichtung gemäß Anspruch 10 gelöst.

Der Kern der vorliegenden Erfindung besteht demzufolge in der Bereitstellung einer sogenannten Schablone oder Führungsschablone, welche beispielsweise bezüglich eines Patientenknochens positioniert, ausgerichtet und am Patienten bzw. am entsprechenden Patientenknochen temporär fixiert werden muss/kann und welche einen Führungsabschnitt zur Führung eines chirurgischen Instruments (oder Implantats) entsprechend der Position und Ausrichtung der Schablone bezüglich des Patientenknochens aufweist. Der Führungsabschnitt ist dabei vorzugsweise an das zu führende chirurgische Instrument angepasst und kann beispielsweise ein Längsschlitz im Fall einer Knochensäge und/oder eine Durchgangsbohrung im Fall eines Knochenbohrers oder einer Fräse als chirurgisches Instrument sein, wobei der entsprechende Führungsabschnitt jeweils an/in der Schablone ausgeformt ist.

Des Weiteren ist bei der erfindungsgemäßen Positionier-/Ausrichtvorrichtung eine optische Markierungseinrichtung (Visiereinrichtung) vorgesehen, mit einer (integrierten) Lichtquelle (einschließlich Stromversorgung), wie z.B. ein Laser oder eine LED (letztere ggf. mit vorgeschalteter Lichtblende, Sammellinse und/oder Prisma), die (nämlich die Markierungseinrichtung) dafür ausgebildet und angepasst ist, (direkt und unmittelbar) auf/an der Schablone temporär in einer vorbestimmten Relativlage und Relativausrichtung zu dieser fixiert zu werden und eine Instrumenten-spezifische geometrische Information wie z.B. eine Schnittlinie (im Fall beispielsweise einer Knochensäge) oder eine Bohrachse (im Fall beispielsweise eines Knochenbohrers/Fräse) auf die Patientenoberfläche zu projizieren.

Soll demnach die (Führungs-) Schablone am Patienten/Patientenknochen fixiert werden, muss zunächst die erfindungsgemäße Markierungseinrichtung an der Schablone in der vorbestimmten Relativlage fixiert und deren Lichtquelle aktiviert werden. Jetzt kann die Schablone ausgerichtet werden, wobei/während die Lichtquelle der Markierungseinrichtung Instrumenten-spezifische Informationen, beispielsweise eine Schnittlinie im Fall einer an eine Knochensäge angepassten Schablone, auf die Oberfläche des Patienten/Patientenknochens im Operationsbereich projiziert.

Sobald die Schablone auf diese Weise korrekt platziert, ausgerichtet und am Patientenkochen fixiert ist, kann die Markierungseinrichtung abgenommen werden.

Bei der Instrumenten-spezifischen geometrischen Information kann es sich im Rahmen der Erfindung insbesondere beispielsweise um einen Punkt und/oder eine Linie und/oder eine Ebene und/oder eine dreidimensionale Darstellung oder Hologramm handeln.

Im Bereich der Orthopädie gab es vor der vorliegenden Erfindung keine Lösungen hinsichtlich einer direkten Visualisierung/Projektion derartiger geometrischer Informationen, insbesondere von Linien und Ebenen, vor Ort, also direkt am/auf Patienten während einer Operation. Unter einem medizintechnischen Instrument im Sinne der Erfindung ist, wie vorstehend bereits angedeutet, insbesondere ein medizintechnisches Bearbeitungsinstrument wie zum Beispiel ein Bohrer, eine Säge, ein Fräser, ein Hobel, ein Schneid-/Verschweiß-Laser, etc. zu verstehen. Es ist aber auch möglich, ein Implantat, insbesondere eine Endoprothese mit der erfindungsgemäßen Vorrichtung auszurichten. Als visualisierte/projizierte geometrische Information können außerdem insbesondere geometrische Informationen des Patienten, wie zum Beispiel Knochenachsen oder Gelenkachsen, Anfangs- und Endpunkte von Knochen, Drehpunkte von Gelenken etc. und/oder geometrische Informationen des Gegenstands, wie insbesondere Arbeitsrichtungen oder -achsen eines chirurgischen Instruments, mit dargestellt werden.

Mittels der Erfindung können eine oder mehrere Ebenen und Achsen gleichzeitig oder zeitlich versetzt und direkt innerhalb des Operationsfeldes visualisiert/projiziert werden. Dadurch können einem Chirurgen oder Operateur die Lage und/oder Ausrichtung der Instrumente und die Lage und/oder Ausrichtung der Schnittführung im Fall einer Knochensäge und damit zum Beispiel ein Knochenschnitt besonders einfach und übersichtlich auf dem Patienten/Patientenknochen dargestellt werden. Im Rahmen der Erfindung kann zum Beispiel ein distaler Tibia-Schnitt in Bezug auf die Tibia-Achse auf dem Patienten/Patientenknochen dargestellt werden. Mit der erfindungsgemäßen Positionier- und/oder Ausrichtvorrichtung ist der Chirurg in einfacher Weise in der Lage, den Einfluss von Fehlstellungen wie Varus oder Valgus und die Neigungsausrichtung auf den Knochenschnitt in Bezug auf die frontale und sagittale Tibia-Achse wahrzunehmen. Außerdem kann zusätzlich die femorale Frontalachse berücksichtigt werden.

Die Projektion oder Visualisierung der geometrischen Informationen kann durch die Erfindung direkt am/auf dem Patienten, insbesondere auf einer knöchernen Oberfläche erfolgen. Die Visualisierung/Projektion von realen Achsen und Ebenen durch die optische Markierungseinrichtung versetzt Chirurgen in die Lage, die Position und Ausrichtung der jeweils verwendeten Instrumente oder Implantate genauer zu bestimmen und dadurch gleichzeitig einige ungenaue Beurteilungen zu vermeiden, die zu suboptimalen Entscheidungen führen könnten.

Die der Erfindung zugrunde liegende Idee kann bei nahezu jeder Operation verwendet werden, bei der eine Ausrichtung einer (Führungs-)Schablone und eines durch diese geführten Instruments/Implantats anhand harter Strukturen wie Knochen erfolgt, zum Beispiel bei Operationen von Hüfte, Knie und Schulter, bei Sprunggelenksprothesen, Osteotomien und auch in der Traumatologie.

Die mit der Erfindung vorgeschlagene Idee geht bei der visuellen Kontrolle von Ausrichtungen und Positionierungen von Instrumenten einen Schritt weiter, indem eine Visualisierung/Projektion von Achsenlinien oder Schnittebenen direkt an dem Patienten ermöglicht wird. Es ist ein besonderer Vorteil der Erfindung, dass sie direkt in ein Integrated Quality System integriert werden kann.

Ein wesentlicher Vorteil der Erfindung ist die Möglichkeit einer schnellen und genauen Überprüfung von Achsen, Positionen und Ausrichtungen mit Hilfe der direkten Visualisierung an Patientenstrukturen, wie Extremitäten, Knochen, etc., wobei die bislang bei bekannten Verfahren und Vorrichtungen, insbesondere bei einer visuellen Beurteilung, inhärente Ungenauigkeit und die Notwendigkeit einer Montage von mechanischen Stangen oder Kontrollplatten vermieden werden können. Weitere Vorteile sind eine Erhöhung der Genauigkeit, eine einfachere Umsetzung der Strategie des Chirurgen, eine Zeitersparnis und eine Verwendbarkeit für eine Vielzahl von Anwendungen und mit unterschiedlichen Instrumenten und Instrumentensets in der Orthopädie.

Ein zusätzlicher Vorteil besteht in einer Verminderung der Anzahl von für einen bestimmten Einsatzfall erforderlichen Ausrichtungs- und Kontrollinstrumenten. Je nach Anwendung können zwischen zwei bis zehn Instrumente eingespart werden. Eine Verwendung einer geringeren Anzahl an Instrumenten ist mit einem geringeren Infektionsrisiko verbunden. Neben einer Verbesserung der Qualitätskontrolle kann außerdem die Vorbereitungs- / Verwendungs- / Wiederaufbereitungszeit der Instrumente vor / während / nach jeder Operation reduziert werden. Dies bedeutet einen Gewinn an Operationszeit, Umlaufzeit und/oder Wiederaufbereitungszeit in der zentralen Instrumentenaufbereitung.

In Anbetracht dessen, dass derzeit die einzigen Alternativen reine visuelle Kontrollen ohne oder mit Hilfe von montierten mechanischen Instrumenten wie Stangen und/oder Kontrollplatten sind, besitzt die Erfindung den Vorteil, dass Achsen und/oder Resektionsebenen direkt auf den Patientenstrukturen im Operationsfeld optisch dargestellt/projiziert werden. Es ist auch wichtig zu bedenken, dass die Erfindung einer Visualisierung auf umwickelten und/oder unebenen Oberflächen zugutekommt, wenn bei der Verwendung bestimmter Instrumente eine mentale Projektion mit der Gefahr eines Parallaxenfehlers erforderlich ist.

Es ist ein besonderer Vorteil der Erfindung, dass die Vorrichtung durch Nutzung der optischen Markierungseinrichtung (Visiereinrichtung) einen besonders kleinen Platzbedarf, nur wenige für eine Verschmutzung anfällige Teile und eine im Wesentlichen größenunabhängige Genauigkeit besitzt. Sie ist daher besonders genau, handlich, verlässlich und günstig, sowohl im Rahmen ihrer Verwendung als auch im Rahmen der Aufbereitung.

Vorteilhafte Ausführungsformen der Erfindung sind in den Unteransprüchen beansprucht und werden nachfolgend näher erläutert.

Gemäß der Erfindung ist die optische Markierungseinrichtung direkt und unmittelbar an bzw. auf der Führungsschablone befestigt/ fixiert bzw. befestigbar/ fixierbar. In anderen Worten ist die optische Markierungseinrichtung (ein Abschnitt derselben) eingerichtet/ angepasst, in Eingriff/ Wirkeingriff mit einem Abschnitt der Führungsschablone gebracht zu werden. Entsprechend ist die Führungsschablone (ein Abschnitt derselben) eingerichtet/ angepasst, in Eingriff/ Wirkeingriff mit einem Abschnitt der optischen Markierungseinrichtung gebracht zu werden. Ein direktes/ unmittelbares Befestigen/ Fixieren bedeutet somit vorliegend insbesondere, dass keine weiteren Bauteile zwischen der optischen Markierungseinrichtung und der Führungsschablone vorgesehen sind. Somit gilt, dass beim bestimmungsgemäßen Einsatz der medizintechnischen Positionier- und/oder Ausrichtungsvorrichtung der vorliegenden Offenbarung ein Abschnitt des Bauteils/ der Komponente "optische Markierungseinrichtung" in Eingriff/ Wirkeingriff mit einem Abschnitt des Bauteils/ der Komponente "Führungsschablone" gebracht ist, so dass die optische Markierungseinrichtung (temporär) an/ auf der Führungsschablone fixiert ist.

Eine Ausführungsform ist dadurch gekennzeichnet, dass die optische Visiereinrichtung zumindest einen Laser aufweist. Dieser kann insbesondere als Laser-Scanner ausgebildet sein. Nach einer weiteren Ausführungsform kann der Laser insbesondere als Linienlaser ausgebildet sein. Darunter ist ein Laser mit einer speziellen Optik zu verstehen, mit der beim Auftreffen des Laserstrahls auf ein Objekt anstatt eines Punktes eine Linie erzeugt wird. Dies kann zum Beispiel durch eine eindimensionale optische Aufweitung des Laserstrahls oder durch eine schnelle Oszillation des Laserstrahls erfolgen. Durch Verwendung eines Lasers kann eine besonders genaue Anzeige der Position und Orientierung der (Führungs-)Schablone und/oder von für eine Bearbeitung relevanten anatomischen Achsen und/oder Richtungen erfolgen. Außerdem benötigt ein Laser nur geringen Bauraum, so dass die Vorrichtung besonders klein ausgebildet werden kann, was ein weites Einsatzgebiet und eine hohe Flexibilität ermöglicht. Das Operationsfeld ist in diesem Fall nicht von der Markierungseinrichtung verdeckt, sondern für den Operateur nahezu uneingeschränkt frei sichtbar und zugänglich.

Eine weitere Ausführungsform der Erfindung ist dadurch gekennzeichnet, dass die Schablone eine Bearbeitungsschablone zur Führung eines medizintechnischen Bearbeitungsinstruments ist, insbesondere eine Sägeschablone, eine Bohrschablone oder eine Frässchablone. Im Rahmen der Erfindung kann die Schablone wie beliebige bekannte Bearbeitungsschablone aus der Medizintechnik ausgebildet sein, ggf. mit dem Unterschied, dass sie bevorzugt eine Koppelstruktur zur lagebestimmten Kopplung mit der optischen Markierungseinheit besitzt. Mittels der Schablone können im Rahmen der Erfindung beliebige medizintechnische Bearbeitungsinstrumente ausgerichtet und insbesondere während der Bearbeitung geführt werden / sein. Beispiele für derartige Instrumente sind Bohrer, Sägen, Fräser, Hobel, Laser, etc.

Vorzugsweise besteht die Koppelstruktur in zumindest einer Ausnehmung, insbesondere in Form eines durchgehenden Lochs oder Sacklochs von beispielsweise rundem, drei-, vier- oder mehreckigem Querschnitt und/oder in Form eines entsprechend geformten Vorsprungs, insbesondere Zapfens. Diese Koppelstruktur der Schablone ist zum Koppeln, Eingreifen und Zusammenwirken mit einer passend geformten Koppelstruktur der optischen Markierungseinrichtung ausgebildet und bestimmt. Es ist von besonderem Vorteil, wenn die Markierungseinheit anwenderseitig lösbar mit der Schablone gekoppelt ist, da dann eine besonders komfortable und anwenderfreundliche Bearbeitung möglich ist, indem zunächst die Schablone mit Hilfe der daran lagebestimmt gekoppelten Markierungseinheit am Patienten angeordnet, in der bestimmungsgemäßen Weise ausgerichtet und nachfolgend fixiert wird und dann die Markierungseinrichtung für die durchzuführende Bearbeitung von der (fixierten) Schablone entkoppelt und entfernt wird.

Alternativ zu der vorstehend beschriebenen Koppelstruktur ist es aber auch möglich, die bereits vorhandenen Führungsabschnitte an der Schablone als schablonenseitige Koppelstruktur für die Markierungseinrichtung zu nutzen, was den Vorteil hat, dass die Markierungseinrichtung in jedem Fall in zu der Schablone korrekter Ausrichtung und Positionierung an der Schablone befestigbar ist.

So kann beispielsweise im Fall einer an Knochensägen angepassten Führungsschablone mit Sägeblatt-Führungsschlitz die Markierungseinrichtung ein Gehäuse mit einer Schwert- oder Flossen-artige Koppelstruktur aufweisen, die in den Führungsschlitz der Schablone nach den Nut- und Feder-Prinzip eingedrückt werden kann. Sie die Flossen-artige Koppelstruktur zudem so ausgebildet, dass sich diese von der Flossenwurzel (nahe zum Einrichtungsgehäuse) ausgehend bis hin zu deren freien Flossenkante verschmälert, kann diese klemmend in den Führungsschlitz der Sageschablone eingesetzt werden - auch bei Führungsschlitzen für unterschiedliche Sägeblattstärken.

Im Fall einer Bohrschablone (nicht näher gezeigt) kann das in dieser ausgebildete Bohrloch als Koppelstruktur für die Markierungseinrichtung dienen, welche in diesem Fall beispielsweise mit einem Dorn-artigen Fortsatz ausgebildet wäre.

Nach einer Ausführungsform der Erfindung kann die optische Markierungseinheit zumindest einen ersten Laserstrahl oder Linienlaserstrahl ausstrahlen. Sie kann insbesondere derart mit der Schablone gekoppelt sein, dass der erste Laserstrahl bzw. Linienlaserstrahl in eine Bearbeitungsrichtung der Schablone gerichtet ist. Auf diese Weise kann der Operateur die Schablone und damit das damit zu verwendende chirurgische Instrument besonders einfach, schnell und genau in der von ihm gewünschten Weise, Lage und Richtung ausrichten.

Nach einer weiteren Ausführungsform der Erfindung kann die optische Markierungseinheit zumindest einen zweiten Laserstrahl oder Linienlaserstrahl ausstrahlen. Sie kann insbesondere derart mit der Schablone gekoppelt sein, dass der zweite Laserstrahl oder Linienlaserstrahl in eine anatomische Achse oder Richtung gerichtet ist. Dies erleichtert dem Operateur die patientenspezifische Orientierung.

Die Markierungseinrichtung kann insbesondere zum Ausstrahlen des ersten und ggf. des zweiten Laserstrahls / Linienlaserstrahls ausgebildet sein. Vorzugsweise sind der erste Laserstrahl / Linienlaserstrahl und ggf. der zweite Laserstrahl / Linienlaserstrahl nichtparallele Laserstrahlen und in einem Winkel zueinander angeordnet, insbesondere orthogonal zueinander ausgerichtet.

Zusammenfassend kann man sagen, dass die Erfindung eine Verwendung von Linienlasern zum Visualisieren/Projizieren von Achsen, Schnittlinien und Orientierungsführungen unmittelbar am Patienten ermöglicht. Eine Materialisierung / Visualisierung geometrischer Information direkt auf einer beispielsweise knöchernen Oberfläche oder eine Visualisierung von realen Achsen und Ebenen durch ein optisches System (die Markierungseinrichtung) kann Chirurgen helfen, die Orientierung und Ausrichtung der verwendeten Geräte genauer zu beurteilen und gleichzeitig zu suboptimalen Entscheidungen führende ungenaue Beurteilungen zu vermeiden.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
- Fig. 1: eine perspektivische Ansicht einer Ausführungsform einer Vorrichtung nach der Erfindung,
- Fig. 2: eine seitliche Ansicht der Ausführungsform der Figur 1,
- Fig. 3: eine perspektivische Ansicht der Ausführungsform der Figur 1 von schräg oben,
- Fig. 4: eine perspektivische Ansicht der Ausführungsform der Figur 1 von schräg unten,

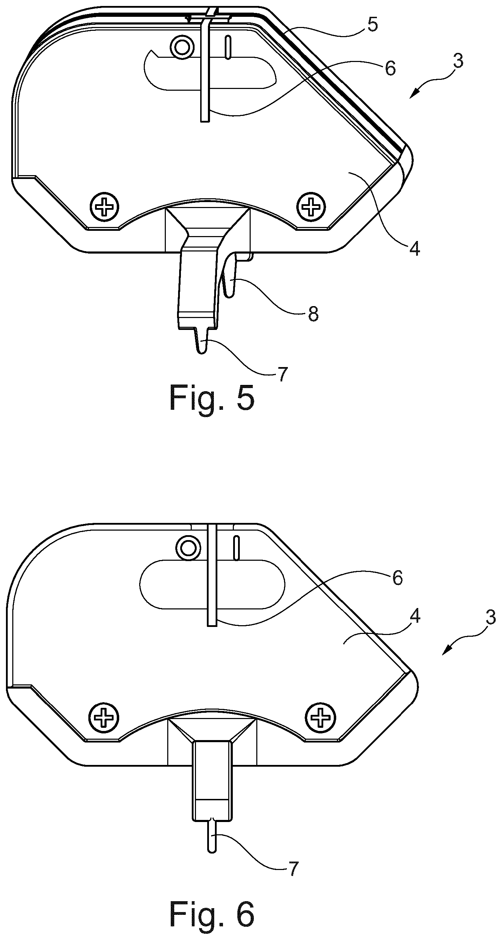

- Fig. 5: eine perspektivische Ansicht einer Markierungseinheit der Vorrichtung der Fig. 1 ohne Schablone,
- Fig. 6: die Markierungseinheit der Fig. 4 aus einer seitlichen Blickrichtung,
- Fig. 7: die Markierungseinheit der Fig. 4 von schräg oben und
- Fig. 8: die Markierungseinheit der Fig. 4 von schräg unten.

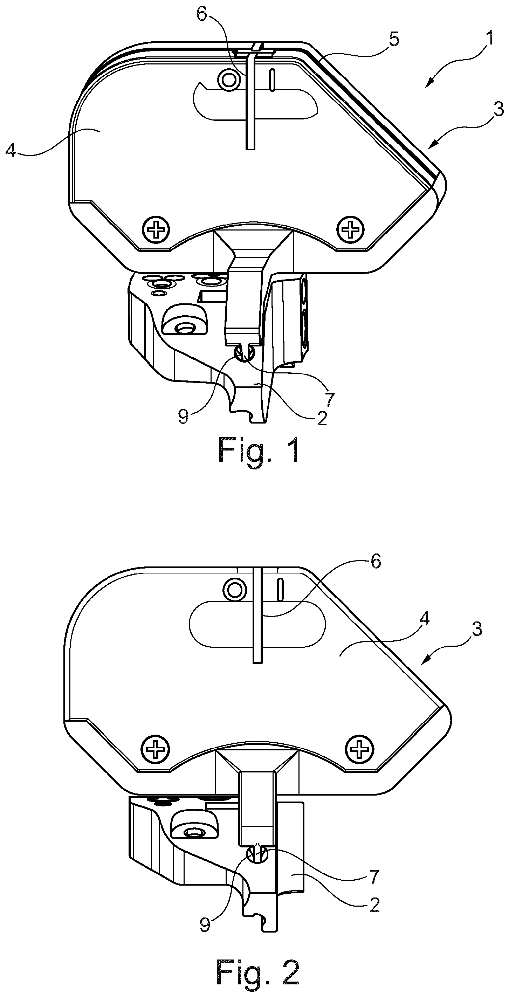

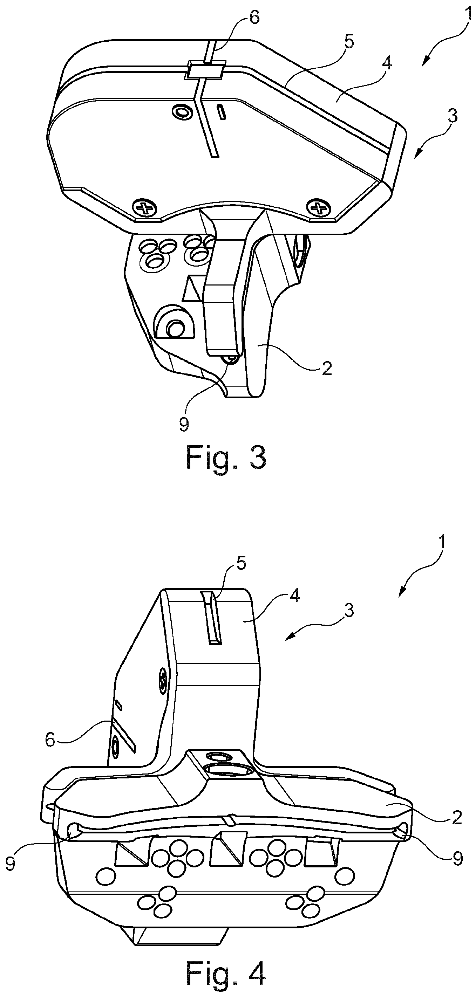

Die Figuren 1 bis 4 zeigen jeweils eine vollständige medizintechnische (Positionier-/Ausricht-)Vorrichtung 1 nach der Erfindung zur Positionierung und/oder Orientierung eines in den Figuren nicht dargestellten chirurgischen Instruments oder Implantats relativ zu einem ebenfalls nicht dargestellten Patienten/Patientenknochen unter direkter Visualisierung/Projektion einer für die Positionierung und/oder Orientierung des Instruments/Implantats geeigneten, Instrumenten-/Implantats-spezifischen geometrischen Information an/auf dem Patienten/Patientenknochen. Im Konkreten zeigen die Fig. 1 - 4 eine Sägeschablone 2 (wie diese bereits aus dem Stand der Technik per se bekannt ist) und eine daran bereits temporär fixierte Markierungseinrichtung 3 als ein bevorzugtes Ausführungsbeispiel der vorliegenden Erfindung.

Die Vorrichtung 1 umfasst demzufolge eine mit dem zu positionierenden und/oder zu orientierenden Knochensäge zusammenwirkende bzw. zum Zusammenwirken geeignete (Führungs-)Schablone 2 und eine mit der Schablone 2 lagebestimmt gekoppelte optische Markierungseinrichtung/-einheit 3 (auch als Visiereinrichtung 3 bezeichnet) zur Visualisierung/Projektion von Sägeblatt-spezifischen geometrischen Informationen, vorliegend beispielsweise einer Schnittlinie.

Die optische Visiereinrichtung 3 weist einen in den Figuren nicht sichtbaren Linienlaser auf, der in einem Gehäuse 4 der Markierungseinrichtung 3 angeordnet ist. Darunter ist ein Laser vorzugsweise mit einer speziellen Optik zu verstehen, mit der beim Auftreffen des Laserstrahls auf ein Objekt (z.B. Patientenknochen) anstatt eines Punktes eine Linie erzeugt wird. Die optische Markierungseinheit 3 strahlt im vorliegenden Beispiel einen ersten Linienlaserstrahl 5 und bevorzugt einen zweiten Linienlaserstrahl 6 aus, deren Ausbreitungsebenen in den Figuren an dem Gehäuse 4 gekennzeichnet sind. Der erste Linienlaserstrahl 5 und der zweite Linienlaserstrahl 6 sind nichtparallel und in einem orthogonalen Winkel zueinander angeordnet und ausgerichtet.

Die Markierungseinrichtung 3 ist derart mit der Schablone 2 gekoppelt, dass der erste Linienlaserstrahl 5 in die Bearbeitungsrichtung der Schablone 2 gerichtet ist. Der zweite Linienlaserstrahl 6 ist in eine anatomische Achse oder Richtung gerichtet. Dadurch können einem Chirurgen oder Operateur die Lage und/oder Ausrichtung des verwendeten und mit der Schablone 2 zu koppelnden Instruments einerseits und andererseits die Lage und/oder Ausrichtung einer Schnittführung und damit zum Beispiel ein Knochenschnitt besonders einfach und übersichtlich unmittelbar/direkt an/auf dem Patienten (-knochen) dargestellt werden. Zum Beispiel kann ein distaler Tibia-Schnitt in Bezug auf die Tibia-Achse dargestellt werden, wobei der erste Linienlaserstrahl 5 die Schnittrichtung und der zweite Linienlaserstrahl 6 die Tibia-Achse markieren und visualisieren.

Zur lagebestimmten Kopplung mit der Schablone 2 besitzt die Markierungseinheit 3 erfindungsgemäß eine Koppelstruktur, hier in Form eines ersten Vorsprungs 7 und eines davon beabstandeten zweiten Vorsprungs 8. Die Schablone 2 weist eine zu den Vorsprüngen 7, 8 passende Ausnehmung 9 in Form einer Durchgangsöffnung 9 auf. Die Markierungseinheit 3 ist einfach in die Schablone einsteckbar und im eingesteckten Zustand lagebestimmt damit gekoppelt. Sie kann besonders einfach anwenderseitig durch Abziehen von der Schablone 2 entfernt werden, so dass letztere für eine Ausrichtung und Führung eines in den Figuren nicht gezeigten Instruments bereit ist.

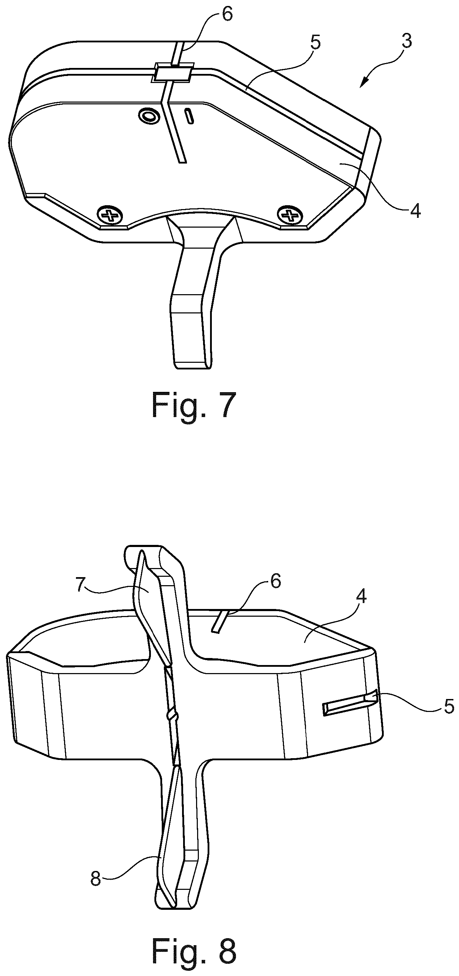

Wie insbesondere aus der Fig. 8 im Konkreten zu entnehmen ist, hat das Gehäuse 4 der Markierungseinrichtung 3 (unterseitig) einen Stütz-/Montagebügel oder Montagefuß bestehend aus einer Führungsschiene, an der sich der zweiteilige, in diesem Ausführungsbeispiel Schwert- oder Flossen-artige Vorsprung 7, 8 ausbildet. D.h. die beiden schwert- oder flossenartigen Vorsprünge sind parallel zueinander auf einer Linie angeordnet. Die Vorsprünge 7, 8 bildet die Markierungsvorrichtungs-seitige Koppelstruktur, die dafür vorgesehen ist, mit der Schablonen-seitigen Koppelstruktur in Eingriff zu kommen.

Da es sich bei der (Führungs-)Schablone 2 im vorliegenden Ausführungsbeispiel um eine Sägeblatt-Schablone handelt, betrifft die Schablonen-seitige Koppelstruktur den Sägeblatt-Führungsschlitz (vorstehend allgemein als Durchgangsöffnung 9 bezeichnet), wie er andeutungsweise in den Fig. 1 und 2 dargestellt ist. Bevorzugt können die Flossen-artigen Vorsprünge 7, 8 in deren jeweiligem Querschnitt dreieckig oder trapezförmig gestaltet sein, sodass diese in den Führungsschlitz 9 (nach dem Nut- und Feder-Prinzip) eingepresst werden können, um so das Gehäuse 4 der Markierungseinrichtung 3 reibschlüssig im Führungsschlitz 9 der Schablone 2 zu halten/fixieren.

Wie ferner insbesondere aus der Fig. 1 zu ersehen ist, befindet sich der zweite Laser/Laserstrahl 6 (in diesem Fall mit schlitzförmiger Lichtaustrittsöffnung) unmittelbar oberhalb der Schablonen-seitigen Koppelstruktur und somit auf einer Ebene mit dem Führungsschlitz der Sägeblatt-Schablone 2. In anderen Worten ausgedrückt verlaufen der Laserstrahl sowie die schwert- oder flossenartigen Vorsprünge 7, 8 im Wesentlichen parallel (ggf. aufeinander zulaufend aber nicht windschief). Im fixierten Zustand der Markierungseinrichtung 3 auf der Schablone 2 lässt sich somit mit Hilfe des zumindest einen (zweiten) Lasers/Laserstrahls 6 eine Linie auf den Patienten/Patientenknochen projizieren, welche sich quasi in Verlängerung zum Führungsschlitz 9 der Schablone 2 erstreckt und somit ein Schnittlinie des Sägeblatts optisch darstellt.

Sobald auf diese Weise die Schablone 2 in korrekter Ausrichtung und Position am Patienten/Patientenknochen fixiert ist, kann die Markierungseinrichtung 3 von der Schablone 2 wieder abgenommen und somit der Führungsschlitz oder Führungsnut 9 für das Sägeblatt (nicht gezeigt) freigegeben werden.

Abschließend sei darauf hingewiesen, dass insbesondere die erfindungsgemäße Markierungseinrichtung 3 als ein Ein-Wege-Produkt ausgestaltet ist. Zu diesem Zweck ist das Gehäuse 4 der Markierungseinrichtung 3 einschließlich der Koppelstruktur 7, 8 aus einem Kunststoff gefertigt (z.B. spritzgegossen), wohingegen die im Gehäuse eingeschlossene Lichtquelle(einschl. Stromquelle) bevorzugt durch einen Laser/eine Lasereinrichtung gebildet wird. Dadurch kann die gesamte Einrichtung 3 schnell und preisgünstig hergestellt werden, sodass sich eine Wideraufbereitung betriebswirtschaftlich nicht rechtfertigt.

### Bezugszeichenliste

- 1: Positionier- und Ausrichtungsvorrichtung
- 2: (Führungs-)Schablone
- 3: optische Markierungseinrichtung
- 4: Gehäuse der Markierungseinrichtung
- 5: erster Laserstrahl / Linienlaserstrahl
- 6: zweiter Laserstrahl / Linienlaserstrahl
- 7: Koppelstruktur der Markierungseinrichtung, (erster) Vorsprung
- 8: Koppelstruktur der Markierungseinrichtung, (zweiter) Vorsprung
- 9: Koppelstruktur der Schablone, Durchgangsöffnung/Führungsschlitz

## Patentansprüche

1. Medizintechnische Positionier- und/oder Ausrichtungsvorrichtung mit einer bezüglich eines Patientenknochens positionier-, ausricht- und am Patientenknochen temporär fixierbaren Führungsschablone (2) für ein chirurgisches Instrument, welche einen Führungsabschnitt (9) zur Führung des chirurgischen Instruments entsprechend der Position und Ausrichtung der Schablone (2) bezüglich des Patientenknochens aufweist, und mit einer optischen Markierungseinrichtung (3), welche zumindest eine Lichtquelle (5, 6) hat und dafür ausgebildet und angepasst ist,
- auf/an der Führungsschablone (2) temporär in einer vorbestimmten Relativlage und Relativausrichtung zu dieser fixiert zu werden, sowie
- eine Instrumenten-spezifische geometrische Information in einer vorbestimmten Relation oder Lagebeziehung zu dem Führungsabschnitt (9) der Führungsschablone (2) auf die Patientenoberfläche oder den Patientenknochen zu projektieren,
**dadurch gekennzeichnet, dass**
die optische Markierungseinrichtung (3) direkt und unmittelbar auf/an der Führungsschablone (2) befestigbar ist, so dass keine weiteren Bauteile zwischen der optischen Markierungseinrichtung (3) und der Führungsschablone (2) vorgesehen sind, und
die optische Markierungseinrichtung (3) ein Gehäuse (4) hat, in welchem die Lichtquelle untergebracht ist und an welchem eine Koppelstruktur (7, 8) ausgeformt ist, die mit einer Koppelstruktur der Schablone (2) in Wirkeingriff bringbar ist.

2. Medizintechnische Positionier- und/oder Ausrichtungsvorrichtung nach Anspruch 1, **dadurch gekennzeichnet, dass** der Führungsabschnitt (9) einen Längsschlitz hat oder ein Längsschlitz ist.

3. Medizintechnische Positionier- und/oder Ausrichtungsvorrichtung nach Anspruch 1, **dadurch gekennzeichnet, dass** der Führungsabschnitt (9) eine Durchgangsbohrung hat oder ist.

4. Medizintechnische Positionier- und/oder Ausrichtungsvorrichtung nach Anspruch 2, **dadurch gekennzeichnet, dass** die Schablonenseitige Koppelstruktur der Längsschlitz (9) ist wohingegen die Koppelstruktur auf Seiten der Markierungseinrichtung (3) ein Montagefuß ist mit einem daran ausgebildeten Flossen-förmigen Vorsprung (7, 8), der dafür ausgebildet und vorgesehen ist, in den Längsschlitz (9) reibschlüssig eingedrückt zu werden.

5. Medizintechnische Positionier- und/oder Ausrichtungsvorrichtung nach Anspruch 1, **dadurch gekennzeichnet, dass** die zumindest eine Lichtquelle (5, 6) einen Linienlaser aufweist.

6. Medizintechnische Positionier- und/oder Ausrichtungsvorrichtung nach Anspruch 1, **dadurch gekennzeichnet, dass** die zumindest eine Lichtquelle (5, 6) als Laser-Scanner ausgebildet ist.

7. Medizintechnische Positionier- und/oder Ausrichtungsvorrichtung nach Anspruch 2, **dadurch gekennzeichnet, dass** die optische Markierungseinrichtung (3) zumindest einen ersten Laserstrahl (4) oder Linienlaserstrahl (4) ausstrahlt und derart mit der Schablone (2) gekoppelt ist, dass der erste Laserstrahl (4) bzw. Linienlaserstrahl (4) in eine Bearbeitungsrichtung der Schablone (4), insbesondere im Wesentlichen in Richtung des Längsschlitzes (9), gerichtet ist.

8. Medizintechnische Positionier- und/oder Ausrichtungsvorrichtung nach Anspruch 7, **dadurch gekennzeichnet, dass** die optische Markierungseinrichtung (3) zumindest einen zweiten Laserstrahl (6) oder Linienlaserstrahl (6) ausstrahlt und derart mit der Schablone (2) gekoppelt ist, dass der zweite Laserstrahl (6) oder Linienlaserstrahl (6) in eine anatomische Achse oder Richtung gerichtet ist.

9. Medizintechnische Positionier- und/oder Ausrichtungsvorrichtung nach Anspruch 8, **dadurch gekennzeichnet, dass** der erste Laserstrahl (5) / Linienlaserstrahl (5) und der zweite Laserstrahl (6) / Linienlaserstrahl (6) in einem Winkel zueinander angeordnet sind, insbesondere orthogonal zueinander ausgerichtet sind.

10. Markierungseinrichtung für eine medizintechnische Positionier- und/oder Ausrichtungsvorrichtung nach einem der vorstehenden Ansprüche 1 bis 9, mit einem Gehäuse (4), in welchem zumindest eine Lichtquelle vorzugsweise in Form eines Lasers untergebracht ist und an welchem außenseitig eine Koppelstruktur (7, 8) vorzugsweise stoffeinstückig ausgebildet ist, **dadurch gekennzeichnet, dass** die Koppelstruktur (7, 8) aus einem Montagefuß besteht, an dem sich ein Flossenartiger Vorsprung (7, 8) ausbildet, dessen freie Flossenkante längs der Abstrahlrichtung der zumindest einen Lichtquelle ausgerichtet ist.

11. Markierungseinrichtung nach Anspruch 10, **dadurch gekennzeichnet, dass** das Gehäuse (4) einschließlich der Koppelstruktur (7, 8) aus Kunststoff vorzugsweise durch Spritzgießen hergestellt ist.

## Claims

1. A medical positioning and/or alignment device comprising a guide template (2) for a surgical instrument which can be positioned, aligned, and temporarily fixed to the patient bone and which has a guide portion (9) for guiding the surgical instrument in accordance with the position and alignment of the template (2) relative to the patient bone, and comprising an optical marking device (3) which has at least one light source (5, 6), **and which** is designed and adapted,
- to be temporarily fixed on/at the guide template (2) in a predetermined relative position and relative orientation thereto, and
- to project instrument-specific geometric information in a predetermined relation or positional relationship to the guide portion (9) of the guide template (2) onto the patient surface or patient bone,
- **characterized in that**
- the optical marking device (3) can be fastened directly and immediately on/to the guide template (2) so that no further components are provided between the optical marking device (3) and the guide template (2), and
- the optical marking device (3) has a housing (4) in which the light source is housed and on which a coupling structure (7, 8) is formed, which can be brought into operative engagement with a coupling structure of the template (2)

2. The medical positioning and/or alignment device according to claim 1, **characterized in that** the guide portion (9) has a longitudinal slot or is a longitudinal slot.

3. The medical positioning and/or alignment device according to claim 1, **characterized in that** the guide portion (9) has or is a through hole.

4. The medical positioning and/or alignment device according to claim 2, **characterized in that** the coupling structure on the template side is the longitudinal slot (9) whereas the coupling structure on the side of the marking device (3) is a mounting base with a fin-shaped projection (7, 8) formed thereon which is adapted and provided to be frictionally pressed into the longitudinal slot (9).

5. The medical positioning and/or alignment device according to claim 1, **characterized in that** the at least one light source (5, 6) comprises a line laser.

6. The medical positioning and/or alignment device according to claim 1, **characterized in that** the at least one light source (5, 6) is designed as a laser scanner.

7. The medical positioning and/or alignment device according to claim 2, **characterized in that** the optical marking device (3) emits at least a first laser beam (4) or line laser beam (4) and is coupled to the template (2) in such a way that the first laser beam (4) or line laser beam (4) is directed in a machining direction of the template (4), in particular substantially in the direction of the longitudinal slot (9).

8. The medical positioning and/or alignment device according to claim 7, **characterized in that** the optical marking device (3) emits at least a second laser beam (6) or line laser beam (6) and is coupled to the template (2) such that the second laser beam (6) or line laser beam (6) is directed in an anatomical axis or direction.

9. The medical positioning and/or alignment device according to claim 8, **characterized in that** the first laser beam (5)/ line laser beam (5) and the second laser beam (6)/ line laser beam (6) are arranged at an angle to each other, in particular aligned orthogonally to each other.

10. A marking device for a medical positioning and/or alignment device according to one of the preceding claims 1 to 9, having a housing (4) in which at least one light source, preferably in the form of a laser, is housed and on the outside of which a coupling structure (7, 8) is formed, preferably in one piece of material, **characterized in that** the coupling structure (7, 8) consists of a mounting base on which a fin-like projection (7, 8) is formed, the free fin edge of which is aligned along the radiation direction of the at least one light source.

11. The marking device according to claim 10, **characterized in that** the housing (4) including the coupling structure (7, 8) is made of plastic, preferably by injection molding.

## Revendications

1. Dispositif médico-technique de positionnement et/ou d'orientation avec un gabarit de guidage (2) pouvant être positionné, orienté par rapport à un os d'un patient et fixé temporairement à l'os du patient pour un instrument chirurgical qui présente une section de guidage (9) pour guider l'instrument chirurgical conformément à la position et l'orientation du gabarit (2) par rapport à l'os du patient, et un appareil de marquage optique (3) qui présente au moins une source de lumière (5, 6) et est conçu et adapté pour
être fixé temporairement sur/au gabarit de guidage (2) dans une position relative et une orientation relative prédéterminée par rapport à celui-ci, ainsi que
- projeter des informations géométriques spécifiques à l'instrument dans une relation ou une position prédéterminée par rapport à la section de guidage (9) du gabarit de guidage (2) sur la surface du patient ou l'os du patient,
**caractérisé en ce que**
l'appareil de marquage optique (3) peut être fixé directement et immédiatement sur/au gabarit de guidage (2), de sorte qu'aucun autre composant supplémentaire n'est prévu entre l'appareil de marquage optique (3) et le gabarit de guidage (2), et
l'appareil de marquage optique (3) présente un boîtier (4) dans lequel la source de lumière est logée et sur lequel est formée une structure de couplage (7, 8) qui peut être mise en prise active avec une structure de couplage du gabarit (2).

2. Dispositif médico-technique de positionnement et/ou d'orientation selon la revendication 1, **caractérisé en ce que** la section de guidage (9) présente une fente longitudinale ou est une fente longitudinale.

3. Dispositif médico-technique de positionnement et/ou d'orientation selon la revendication 1, **caractérisé en ce que** la section de guidage (9) présente ou est un alésage traversant.

4. Dispositif médico-technique de positionnement et/ou d'orientation selon la revendication 2, **caractérisé en ce que** la structure de couplage côté gabarit est la fente longitudinale (9), tandis que la structure de couplage côté appareil de marquage (3) est un pied de montage avec une saillie (7, 8) en forme d'aileron formée sur celui-ci, qui est conçue et prévue pour être enfoncée par friction dans la fente longitudinale (9).

5. Dispositif médico-technique de positionnement et/ou d'orientation selon la revendication 1, **caractérisé en ce que** la au moins une source de lumière (5, 6) présente un laser linéaire.

6. Dispositif médico-technique de positionnement et/ou d'orientation selon la revendication 1, **caractérisé en ce que** la au moins une source de lumière (5, 6) est conçue en tant que scanner laser.

7. Dispositif médico-technique de positionnement et/ou d'orientation selon la revendication 2, **caractérisé en ce que** l'appareil de marquage optique (3) émet au moins un premier faisceau laser (4) ou faisceau laser linéaire (4) et est couplé au gabarit (2) de sorte que le premier faisceau laser (4) ou le faisceau laser linéaire (4) est dirigé dans une direction de traitement du gabarit (4), en particulier sensiblement en direction de la fente longitudinale (9).

8. Dispositif médico-technique de positionnement et/ou d'orientation selon la revendication 7, **caractérisé en ce que** l'appareil de marquage optique (3) émet au moins un second faisceau laser (6) ou faisceau laser linéaire (6) et est couplé au gabarit (2) de sorte que le second faisceau laser (6) ou faisceau laser linéaire (6) est dirigé dans un axe ou une direction anatomique.

9. Dispositif médico-technique de positionnement et/ou d'orientation selon la revendication 8, **caractérisé en ce que** le premier faisceau laser (5)/faisceau laser linéaire (5) et le second faisceau laser (6)/faisceau laser linéaire (6) sont disposés l'un par rapport à l'autre selon un angle, en particulier sont orientés orthogonalement l'un par rapport à l'autre.

10. Appareil de marquage pour un dispositif médico-technique de positionnement et/ou d'orientation selon l'une quelconque des revendications 1 à 9 précédentes, avec un boîtier (4) dans lequel au moins une source de lumière, de préférence sous forme d'un laser, est logée, et sur lequel une structure de couplage (7, 8) est conçue côté extérieur, de préférence en un seul matériau, **caractérisé en ce que** la structure de couplage (7, 8) est constituée d'un pied de montage sur lequel se forme une saillie (7, 8) en forme d'aileron dont le bord d'aileron libre est orienté le long de la direction de rayonnement de la au moins une source de lumière.

11. Appareil de marquage selon la revendication 10, **caractérisé en ce que** le boîtier (4), structure de couplage (7, 8) incluse, est fabriqué en matière plastique, de préférence par un moulage par injection.
